(19) **Europäisches Patentamt / European Patent Office / Office européen des brevets**

(11) **EP 1 958 689 A1**

(12) **EUROPEAN PATENT APPLICATION**

(43) Date of publication:
**20.08.2008 Bulletin 2008/34**

(51) Int Cl.:
**B01J 19/00** *(2006.01)* **C12Q 1/68** *(2006.01)*

(21) Application number: **08002295.7**

(22) Date of filing: **07.02.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **13.02.2007 KR 20070014935**

(71) Applicant: **Samsung Electronics Co., Ltd.**
**Suwon-si, Gyeonggi-Do (KR)**

(72) Inventors:
• **Kim, Won-Sun**
  **Suwon-si**
  **Gyeonggi-do (KR)**

• **Chi, Sung-Min**
  **Hwaseong-si**
  **Gyeonggi-do (KR)**
• **Hah, Jung-Hwan**
  **Toyonaka City,**
  **Osaka Prefacture, 560-0083 (JP)**
• **Kim, Kyoung-Seon**
  **Suwon-si**
  **Gyeonggi-do (KR)**

(74) Representative: **Kuhnen & Wacker**
**Patent- und Rechtsanwaltsbüro**
**Prinz-Ludwig-Strasse 40A**
**85354 Freising (DE)**

(54) **Microarray and method of producing the same**

(57)    A microarray includes a substrate (110), a plurality of fine particles (120) disposed on the substrate (110) at regular intervals, and a plurality probes (130) coupled with the fine particles.

**Description**

## BACKGROUND OF THE INVENTION

### 1. Technical Field

The present disclosure relates to a microarray and more particularly, to a microarray with improved light detection and a method of producing the same.

### 2. Description of the Related Art

As the genome project advances, genome nucleotide sequences of a variety of organisms have been found, which has thereby increased the interest in using microarrays. The microarray is extensively used to perform gene expression profiling and genotyping to detect mutation and polymorphism such as, for example, single nucleotide polymorphism (SNP), to analyze protein and peptide, to perform screening of potential drugs, and to develop and produce new drugs.

The microarray includes a plurality of probes that is fixed to a substrate. The probes are directly fixed through spotting, or being synthesized in situ by using photolithography to perform the fixing. The probes are made of a substance capable of being mixed with a target substance. If a target substance containing a fluorescent substance is mixed with a probe, the fluorescent substance is to remain on the microarray. The fluorescent substance is excited to emit light so as to determine the presence or non-presence of mixing as well as the degree thereof.

Like other microelectronic fields, the recent interest in microarrays comes from the fact that more information can be obtained from smaller substrates. For this to occur, the amount of fixed probes per unit area should be increased and a high rate of light detection in relation to mixed amounts should also obtained. Significant effort has been made to achieve this, but no satisfactory method satisfying the above-mentioned requirements has been suggested in the conventional art.

## SUMMARY OF THE INVENTION

Exemplary embodiments of the present invention provide a microarray with an integrated probe density and high rate of light detection.

Exemplary embodiments of the present invention provide a method of producing a microarray with an integrated probe density and a high rate of light detection.

In accordance with an exemplary embodiment of the present invention, a microarray is provided. The microarray includes a substrate, a plurality of fine particles disposed on the substrate at regular intervals, and a plurality of probes coupled with the fine particles.

In accordance with an exemplary embodiment of the present invention, a method of producing a microarray is provided. The method includes providing a substrate, disposing a plurality of fine particles on the substrate so that the fine particles are disposed at regular intervals, and coupling a plurality of probes with the fine particles.

Details of other exemplary embodiments are included in the detailed description and drawings.

## BRIEF DESCRIPTION OF THE DRAWINGS

Exemplary embodiments of the present invention can be understood in more detail from the following description taken in conjunction with the attached drawings in which:

FIG. 1 is a plan view of a microarray according to an exemplary embodiment of the present invention;
FIG. 2 is a cross-sectional view of the microarray of FIG. 1 taken along the line II-II';
FIG. 3 is an enlarged view of nanoparticles according to an exemplary embodiment of the present invention;
FIG. 4 is a plan view of a microarray according to an exemplary embodiment of the present invention;
FIGS. 5 to 9 are cross-sectional views of microarrays according to an exemplary embodiment of the present invention;
FIGS. 10 to 15 are cross-sectional views illustrating each production process of the microarray shown in FIG. 2;
FIG. 16 is a cross-sectional view illustrating each production process of the microarray shown in FIG. 6; and
FIGS. 17 to 19 are cross-sectional views illustrating each production process of the microarray shown in FIG. 7.

## DESCRIPTION OF THE EXEMPLARY EMBODIMENTS OF THE INVENTION

The present invention may, however, be embodied in many different forms and should not be construed as being limited to the exemplary embodiments set forth herein.

Spatially relative terms, such as "below", "beneath", "lower", "above", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. Like reference numerals refer to like elements throughout the specification.

Exemplary embodiments of the present invention will be described in detail with reference to the accompanying drawings hereinafter.

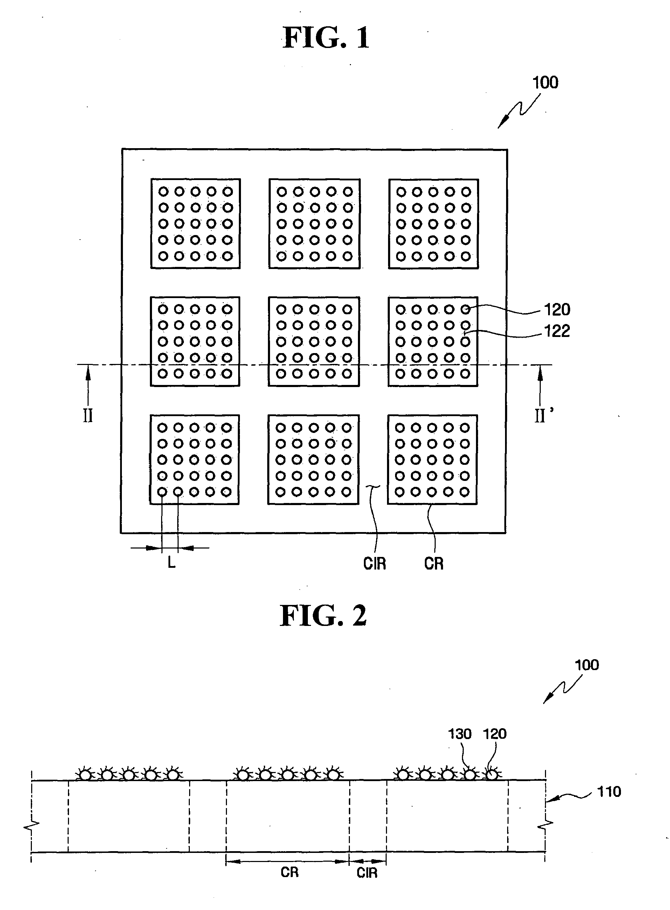

FIG. 1 is a plan view of a microarray according to an exemplary embodiment of the present invention.

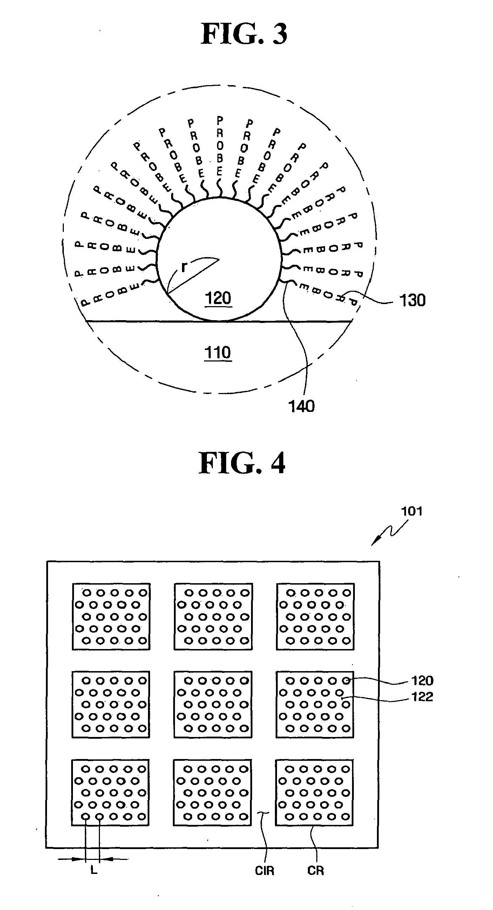

FIG. 2 is a cross-sectional view of the microarray of FIG. 1 taken along the line II-II (. FIG. 3 is an enlarged view of nanoparticles according to an exemplary embodiment of the present invention. FIG. 4 is a plan view of a microarray according to another an exemplary embodiment of the present invention.

With reference to FIGS. 1 to 3, a microarray 100, according to an exemplary embodiment of the present invention, includes a substrate 110, a plurality of fine particles 120 that are disposed on the substrate, and a plurality of probes 130 that are coupled with the fine particles 120.

The substrate 110 may be, for example, a flexible or rigid substrate. Examples of the flexible substrate may include but are not limited to membranes or plastic films made of nylon or nitrocellulose. Examples of the rigid substrate may include but are not limited to a silicon substrate or a transparent glass substrate of soda-lime glass. In the case of the silicon substrate or the transparent glass substrate, nonspecific bonding hardly occurs during a hybridization process. Additionally, the silicon substrate or the transparent glass substrate may be beneficial because the process producing various thin films or photolithography process typically used to produce semiconductor devices or LCD panels can be applied to them without modification.

The substrate 110 may include a probe cell region (CR) and a probe cell isolation region (CIR). The probe cell region (CR) and the probe cell isolation region (CIR) of the substrate 110 may not be physically distinguishable on their own but distinguished according to how the probe 130 is disposed, For example, the probe 130 is disposed in the probe cell region (CR), but not in the probe cell isolation region (CIR). The probe cell region (CR) may be divided by the probe cell isolation region (CIR) into a plurality of regions, that is, the probe cell region (CR) is defined by the probe cell isolation region (CIR) which surrounds it.

A plurality of fine particles 120 is disposed in the probe cell region (CR) of the substrate 110. The fine particles 120 may have, for example, a spherical or oval shape physically. In the case that the fine particle 120 has a physically spherical shape, the fine particle 120 may be a nanoparticle or a microparticle about 50 to about 10,000 nm in diameter (2r). In the case that the fine particle 120 is roughly oval, the fine particle 120 may be a nanoparticle or a microparticle with a major axis of about 50 to about 10,000 nanometers (nm). For example, each fine particle 120 may have an identical diameter (2r) or major axis. The fine particles 120 may be made of, for example, inorganic substances such as silicon oxides (for example, $SiO_2$) or titanium oxides ($TiO_2$), methyl methacrylate, styrene, dimethyl siloxane, vinyl alcohol, hydroxy methacrylate, or a polymer or copolymer thereof. Furthermore, the fine particle 120 may be made of two or more substances out of the above-mentioned substances.

The fine particles 120 may be, for example but not limited to, disposed to form a single layer. Furthermore, the fine particles 120 are disposed at regular intervals (L). In connection with this, the interval (L) means the distance between the centers of adjacent fine particles 120. By this reason, the fine particles 120 are unidirectionally disposed so that the interval (L) between adjacent fine particles 120 can be constant. For example, as shown in FIG. 1, the fine particles may be disposed in a checkerboard form where perfect square unit structures are regularly arranged. In this case, lengthwise and widthwise intervals (L) between adjacent fine particles 120 are constant.

As shown in FIG. 4, in a microarray 101 obtained from another exemplary embodiment, regular triangle unit structures may be regularly arranged. In this case, the intervals (L) between adjacent fine particles 120 in the width direction or in a direction that has an angle of about $\pm$ 60° degrees to the width direction are constant.

Furthermore, the fine particles 120 may be spaced out at regular intervals (L). The spacing of fine particles means that adjacent fine particles 120 are not in contact with one another and space is provided between the adjacent fine particles. Therefore, as shown in FIGS. 1 and 4, the interval (L) between adjacent fine particles 120 is larger than the sum total of radii (r) of adjacent fine particles 120.

The fine particles 120 that are spaced out at regular intervals (L) may constitute a photonic crystal structure. That is, as shown in FIG 1 or 4, the repeated structure of the fine particles 120 and the space 122 which have a predetermined refractive index may have a connection with the photonic crystal structure which amplifies the intensity of light passing through the fine particles. In this connection, the space 122 may have a refractive index different from that of the fine particles 120. For example, the space may be in a vacuum. Alternatively, air may occupy the space 122. The minimum width of the space 122, that is, the minimum distance between adjacent fine particles 120, may be about 10,000 nm or less.

The fine particles 120 that constitute the photonic crystal structure may be disposed to make the distance (L) between the centers of the fine particles 120 fall in the range between about 150 to about 20,000 nm. For example, the distance (L) between the centers of adjacent fine particles 120 that constitute the photonic crystal structure may be calculated using the following Equation.

[Equation]

$$L = \frac{\lambda}{2}n$$

In the above Equation, L is the distance between the centers of the adjacent fine particles, λ is the wavelength of detected fluorescent light, and n is the natural number.

In the case that the above-mentioned Equation is satisfied, the wavelength of detected light becomes fully amplified. Therefore, the distance (L) between the centers of adjacent fine particles 120 may be determined according to the wavelength (λ) of detected fluorescent light. The wavelength (λ) of detected fluorescent light may be, for example but not limited to, about 300 to about 600 nm. Meanwhile, the distance (L) between the centers of adjacent fine particles 120 does not strictly depend on the above-mentioned Equation. However, the Equation is just an example to show that the closer the distance gets to the Equation, the better degree of amplification can be obtained.

Each of the fine particles 120 may have a functional group capable of being coupled with the probe 130 on the surface thereof, and may be directly coupled with a plurality of probes 130 through the functional group or through the functional group and the linker 140.

The probe 130 may be, for example, an oligomer probe. Here, the term "oligomer" means the polymer that is made of two or more monomers covalent bonded to one another. The oligomer may include about 2 to about 500 monomers. For example, the oligomer may include about 5 to about 300 monomers, e.g., about 5 monomers to about 100 monomers. However, in the oligomer of exemplary embodiments of the present invention, the molecular weight is not limited to the above-mentioned numerical value, but may include any oligomer known in the art.

Examples of the monomer of the oligomer probe may include but are not limited to nucleosides, nucleotides, amino acids, or peptides.

Nucleosides and nucleotides may include but are not limited to a known purine or pyrimidine base, or include methylated purine or pyrimidine, or acylated purine or pyrimidine. Examples of the base may include but are not limited to adenine (A), guanine (G), thymine (T), cytosine (C), or uracil (U). Furthermore, nucleosides and nucleotides may include but are not limited to known ribose or deoxyribose saccharides, or include but are not limited to modified saccharides in which one or more hydroxyl groups are substituted by halogen atoms or aliphatics or to which the functional group such as ether or amine is bonded.

The amino acid may be, for example, an L-, D-, or nonchiral-type amino acid which is found in nature. Alternatively, the amino acid may be a modified amino acid or an analog of the amino acid.

The peptide is a compound made by, for example, amide bonding between a carboxyl group of an amino acid and an amino group of another amino acid.

Therefore, the probe (oligomer probe) 130 may be made of, for example, two or more nucleosides, nucleotides, amino acids, and peptides.

In the case that the linker 140 mediates to couple the fine particles 120 with the probe 130, the linker 140 may be made of substances containing both functional groups capable of being coupled with the fine particles 120 and functional groups capable of being coupled with the probe 130. The linker 140 may work to provide a spatial margin necessary for mixing and for this, the length of the linker 140 may be, for example but not limited to, about 6 to about 50 atoms.

Meanwhile, the fine particles 120 may remain in the probe cell isolation region (CIR) of the substrate in the exemplary embodiments of the present invention. In this case, however, the probe 130, for example, should not be coupled with the fine particles 120 in the probe cell isolation region (CIR).

As described above, the fine particle 120 is formed to have the sphere or oval shape and the probe 130 is coupled with the functional group that is provided on the surface of the fine particle 120. Thus, the probe 130 may be coupled with a portion of the surface of the fine particle 120 other than the surface facing the substrate 110. That is, as shown in FIG 3, the coupling of the probe is performed upward and horizontally with respect to the substrate 110. In addition, the coupling of the probe is performed at an inclined direction. That is, as the three-dimensional fine particle 120 having the sphere or oval shape has a surface area larger than that of the two-dimensional structure, the number of coupled probes 130 per unit area may be increased. Accordingly, it can be seen that the integration of probe 130 may be improved.

In addition, as described in the above, as the fine particles 120 constitute the photonic crystal structure, the intensity of detected light may increase according to the degree of mixing. Therefore, mixing can be readily detected even when a relatively small amount of probe 130 is integrated.

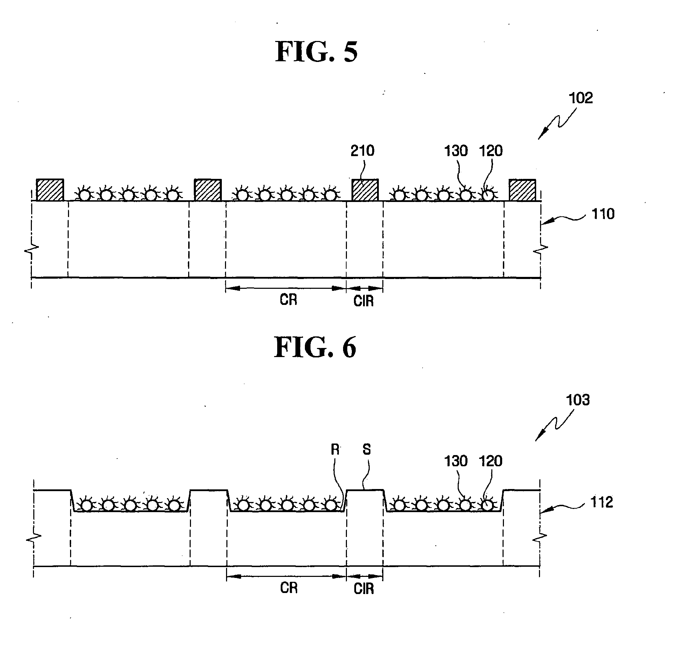

Microarrays according to the other exemplary embodiments of the present invention will be described hereinafter. A description may be omitted or briefly given of the same structure and constitution as the embodiments of FIGS. 1 to 3 in the present invention, and a difference thereof will be mainly described. FIGS. 5 to 9 are cross-sectional views of microarrays according to the other exemplary embodiments of the present invention.

First, the microarray 102 shown in FIG. 5 is different from the microarray 100 of FIG. 2 in that a first mask pattern 210 is formed in the probe cell isolation region (CIR) of the substrate 110. That is, the probe cell region (CR) and the probe cell isolation region (CIR) are not apparently distinguishable only with the shape of the substrate 110 but the probe cell region (CR) can be physically defined when the first mask pattern 210 is formed in the probe cell isolation region (CIR). The first mask pattern 210 may cover either the entire probe cell isolation region (CIR) or a portion of the probe cell isolation region (CIR). That is, the first mask pattern 210 according to the exemplary embodiments of the present invention may be designed to expose a portion of the probe cell isolation region (CIR) of the substrate.

The first mask pattern 210 may be made of, for example but not limited to ; a silicon oxide layer, a silicon nitride layer, a silicon oxynitride layer or a photoresist layer in the subsequent process as long as interaction does not occur.

The microarrays according to some exemplary embodiments of the present invention may include a recess pattern on the surface of the substrate. For example, as shown in FIG. 6, the recess pattern (R) that is included in the microarray 103 may be disposed in the probe cell region (CR). The surface (s) of the substrate 112 which is relatively protruding from the recess pattern (R) is disposed in the probe cell isolation region (CIR). With the above-mentioned structure, the probe cell region (CR) and the probe cell isolation region (CIR) are physically distinguishable from each other. However, the present exemplary embodiment is not limited to the case where the probe cell region (CR) corresponds to the recess pattern (R) and the probe cell isolation region (CIR) to the protruding surface (s) of the substrate 112. For example, like the embodiment of FIG. 5, the protruding surface (s) of the substrate 112 occupies a portion of the probe cell isolation region (CIR) and the remaining portion of the probe cell isolation region (CIR) may be made of the recess pattern (R).

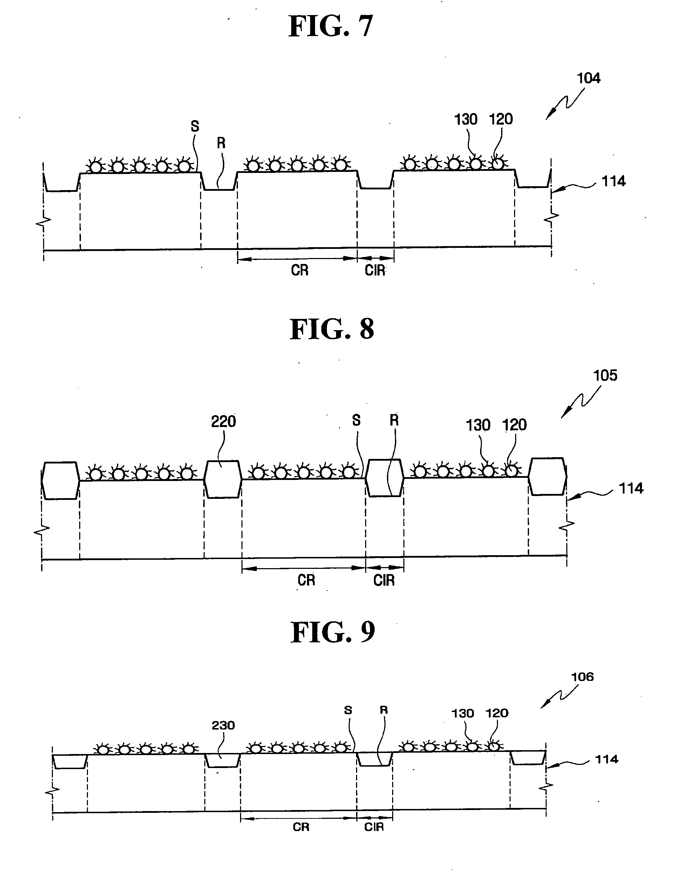

The microarray 104 of the embodiment of FIG. 7 is substantially identical to that of the embodiment of FIG. 6 in that the substrate 114 includes the recess pattern (R) on the surface (s) thereof. However, unlike the embodiment of FIG. 6, the recess pattern (R) is disposed in the probe cell isolation region (CIR) and the protruding surface (s) of the substrate 114 in the probe cell region (CR), thereby making the probe cell isolation region (CIR) including the recess pattern (R) physically distinguishable from the probe cell region (CR).

Unlike the embodiment of FIG. 7, the embodiments of FIGS. 8 and 9 illustrate the case where isolation layers 220 and 230 covering the recess patterns (R) are provided in the probe cell isolation regions (CIR), that is, as shown in FIG. 8, the microarray 105 obtained from the exemplary embodiments of the present invention may include the isolation layer 220 which covers the recess pattern (R) by protruding upward from the surface (s) of the substrate 114. The isolation layer 220 may be a local oxidation of silicon (LOCOS) layer. As shown in FIG. 9, the microarray 106 obtained from other exemplary embodiments of the present invention may include the isolation layer 230 which covers the recess pattern (R) and of which surface is leveled to the surface (s) of the substrate 114. The isolation layer 230 may be an STI layer. The isolation layers 220 and 230 are not limited to those described herein.

A method of producing the microarrays will be described hereinafter.

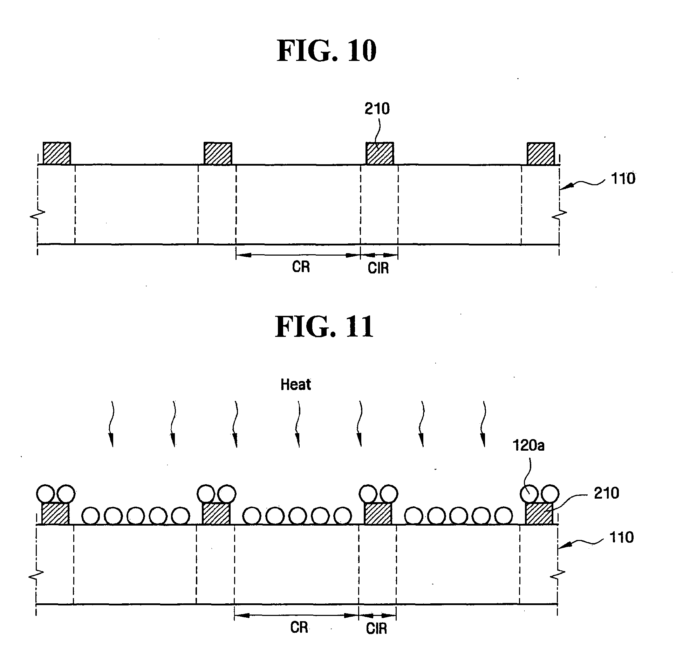

First, the method of producing the microarrays shown in FIG. 2 shall be described. FIGS. 10 to 15 are cross-sectional views illustrating each production process of the microarray shown in FIG. 2.

With reference to FIG. 10, the substrate 110 is provided to form the first mask pattern 210 on the substrate 110. The first mask pattern 210 covers the probe cell isolation region (CIR) and is formed in the way to expose the probe cell region (CR). The first mask pattern 210 may be made of, for example, silicon oxide layer, silicon nitride layer, silicon oxynitride layer, or photoresist layer.

With reference to FIG. 11, fine particle precursors 120a are provided on the substrate 110, which may be made of, for example, inorganic substances such as silicon oxides (for example, $SiO_2$) or titanium oxides ($TiO_2$), methyl methacrylate, styrene, dimethyl siloxane, vinyl alcohol, hydroxy methacrylate, or polymer or copolymer thereof. Furthermore, the fine particle precursors 120a may be made of two or more substances out of the above-mentioned substances.

For example, the fine particle precursors 120a may have a spherical shape and identical diameter. For example, they may be about 150 to about 20,000 nm in diameter. However, cases where the fine particle precursors 120a are an oval shape are not excluded from the present exemplary embodiment.

For example, the fine particle precursors 120a may be dispersed in a solvent such as alcohol to be provided as a dispersion solution. The dispersion concentration may be, for example, about 0.1 to about 10%. In the case where the fine particle precursors 120a are provided as a dispersion solution, for example, a spin coating process or a slit coating process may be used. In the case where the spin coating process is performed, the coating may be performed at the speed of about 200 to about 1,000 rpm.

For example, the fine particle precursors 120a should be provided as a dispersion solution under the condition that they are disposed to form a single layer. Sometimes, the spin coating is useful for the control of the disposal of the fine particle precursors 120a in a single layer.

The next step is to agglomerate the fine particle precursors 120a through annealing. The annealing temperature may be, for example, about 300 to about 1,000°C. The annealing time may be, for example, about 5 to about 24 hours. In the present exemplary embodiment of the present invention, the annealing temperature and time may be about 500°C and about 12 hours, respectively.

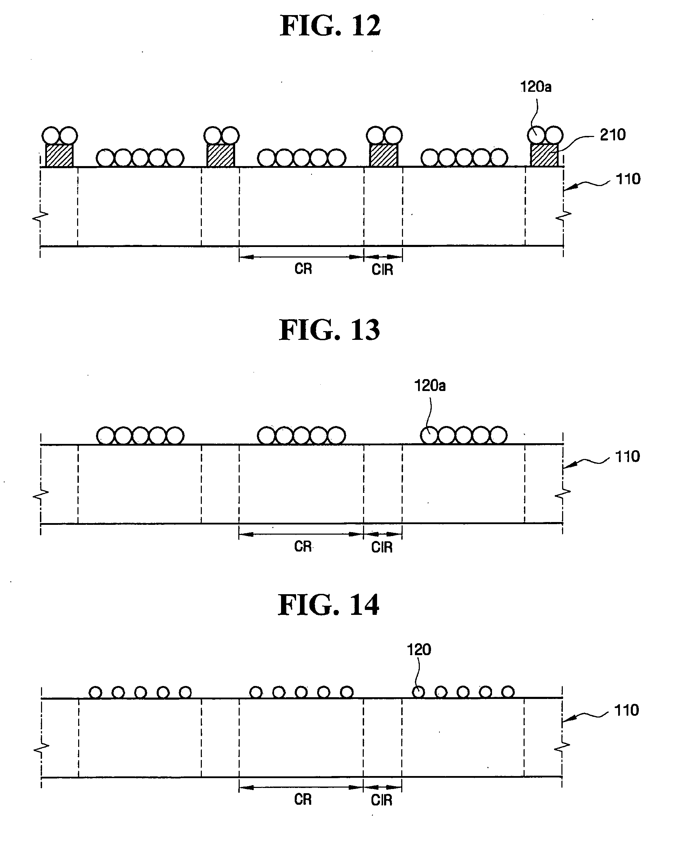

With reference to FIG. 12, the fine particle precursors 120a agglomerated through annealing are not spaced out at certain intervals but instead, in close contact with each other. In the exemplary embodiments of the present invention, the distance between the centers of adjacent agglomerated fine particle precursors 120a is about two times the radius of the fine particle precursors 120a. Therefore, in the case that the fine particle precursors 120a are identical in size, the distance between the centers of adjacent fine particle precursors 120a is constant.

In some exemplary embodiments, the fine particle precursors 120a of the probe cell region (CR) agglomerate towards the center of the probe cell region (CR) between the first mask patterns 210. Therefore, the fine particle precursors 120a may not be present in the periphery of the first mask patterns 210. As described in the above, when distinguishing the probe cell region (CR) from the probe cell isolation region (CIR) by the presence or non-presence of probes coupled with fine particles, the periphery of the first mask pattern 210 where the fine particle precursors 120a are not present may be considered the probe cell isolation region (CIR). That is, the probe cell isolation region (CIR) may include an exposed portion not being covered with the first mask pattern 210.

With reference to FIG. 13, optionally, the first mask pattern 210 is removed. The removal of the first mask pattern 210 may be performed in different ways according to the type of substance that constitutes the first mask pattern 210. For example, in the case where the first mask pattern 210 is made of silicon oxide layer, silicon nitride layer, or silicon oxynitride layer, the removal may be performed using a wet etching process. In the case that the first mask pattern 210 is made of a photoresist layer, the first mask pattern 210 may be removed using, for example, an ashing process or a strip process. For example, the removal of the first mask pattern 210 should be performed under the condition that the fine particle precursors 120a provided in the probe cell region (CR) are not attacked. Therefore, in some exemplary embodiments, it is recommended that the first mask pattern 210 should be made of the photoresist layer and removed using, for example, the ashing process or the strip process, which may entail the removal of the fine particle precursors 120a which may be disposed on the first mask pattern 210 at the step of FIG. 11.

Meanwhile, to keep the first mask pattern 210 on the resulting structure like the microarray 102 of FIG. 5, the removal process of the first mask pattern 210 may be omitted.

With reference to FIG. 14, the sizes of the fine particle precursors 120a are reduced to form the fine particles 210. The sizes of the fine particle precursors 120a may be reduced using, for example, isotropy etching. Examples of the isotropy etching may include but are not limited to wet etching using hydrogen fluoride (HF). The size of the fine particle precursor 210a is reduced in all directions due to the isotropy etching. In connection with this, the shape of the fine particle precursor 120a is not changed. That is, in the case of when the fine particle precursor 120a has the sphere shape, the fine particle 120 has the sphere shape. In the case of when the fine particle precursor 120a has the oval shape, the fine particle 120 has the oval shape. However, in a specific process condition of isotropy etching, the conversion of the fine particle precursors 120a into fine particles 120 may cause change in their shapes. For example, the spherical shape of the fine particle precursors 120a may become elliptical fine particles 120 after isotropy etching. Given that, the fine particle 120 should have, for example, a spherical shape. The shape of the fine particle precursor 120a may be set in consideration of any shape change that may occur in the isotropy etching process.

The shape of the fine particle 120 that is formed through the size reduction of the fine particle precursor 120a may be, for example, a sphere or oval having the diameter or major axis of about 50 to about 10,000 nm. For example, the sizes of the fine particles 120 be the same as each other. The fine particles 120 that are reduced in size do not contact with the neighboring fine particles 120 but are spaced apart from each other. In the case where the distances between the centers of the adjacent fine particle precursors 120a are the same as each other, the distances between the centers of the adjacent fine particles 120 that are reduced in size are the same as each other, the fine particles 120 are disposed at regular intervals. Therefore, the distance (L) between the centers of the adjacent fine particles 120 may be substantially the same as the diameter of the fine particle precursor 120a. That is, the distance (L) between the centers of the adjacent fine particles may be about 150 to about 20,000 nm.

Then, air, which has a refractive index different from that of the fine particles 120 occupies the space between the particles. Such fine particles 120 and space structure constitute the photonic crystal structure which amplifies the intensity of detected light. The minimum width of space, that is, the minimum distance between adjacent fine particles, may be about 10,000 nm or less. Meanwhile, in the case that the solvent coming from the dispersion solution of the find particle precursors 120a selectively remains on the substrate 110 in the present process or the subsequent process, the remaining solvent may be removed using, for example, the spin dry process.

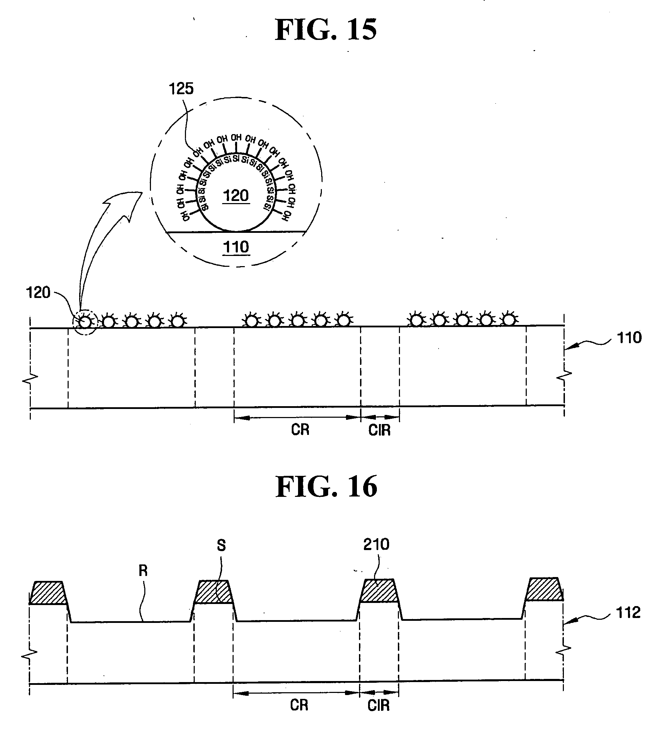

With reference to FIG. 15, the surface of the fine particles 120 is activated to expose the functional groups 125 that are capable of being coupled with the probes thereon. Examples of the surface activation process may include but are not limited to the cleaning treatment of the fine particles 120 using a sulfuric acid ($H_2SO_4$) for about 1 hour. For example, in the case that the fine particles 120 are made of silicon oxide, silanol (-SiOH) groups may be exposed on the surface of the fine particles 120 during the cleaning using, for example, the sulfuric acid.

With reference to FIG. 2 here again, the probes 130 are coupled with the fine particles 120, the functional groups 125 of which are exposed. The probes 130 may be coupled by means of the in-situ synthesis process using monomers for probes. Furthermore, coupling of the probes may be mediated by the linker. As the synthesis and coupling of the probes can be easily inferred from the processes known in the related art, a detailed description thereof shall be omitted. When the probes are coupled as described above, the microarray shown in FIG. 2 may be obtained.

FIG. 16 is a cross-sectional view illustrating each production process of the microarray shown in FIG. 6.

The present exemplary embodiment is substantially identical with that of FIG. 10, with the exception of the steps after the first mask pattern 210 is formed on the substrate. Subsequently, with reference to FIG. 16, the exposed surface of the substrate 110 is etched using the first mask pattern 210 as the etching mask. Due to the etching, the recess pattern (R) on the surface (s) of the substrate 112 is formed in the probe cell region (CR) of the substrate 112. The subsequent processes are substantially identical to those of the embodiments of FIGS. 11 to 15, except that the recess pattern (R) is provided in the probe cell region (CR) of the substrate 110. Therefore, a detailed description thereof shall be omitted.

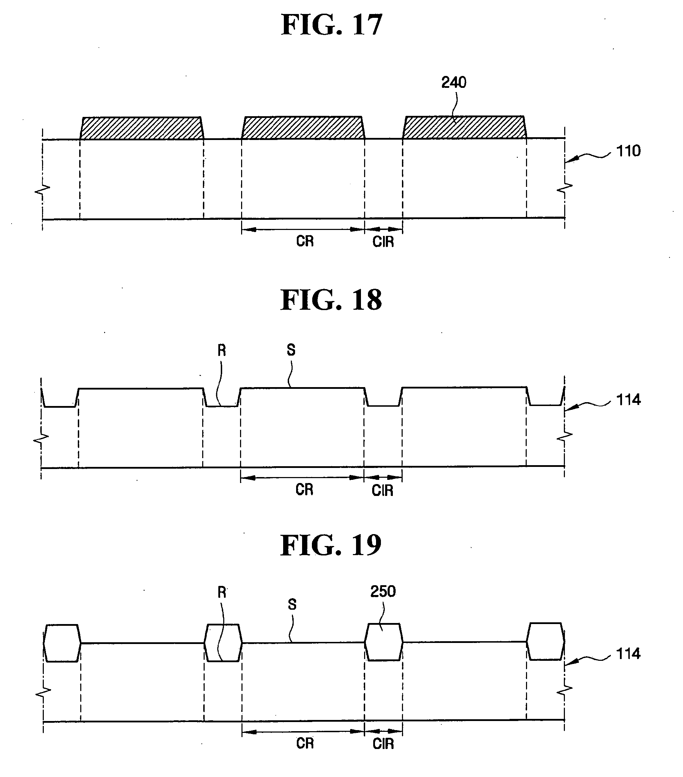

FIGS. 17 to 19 are cross-sectional views illustrating each production process of the microarray shown in FIG. 7.

With reference to FIG. 17, first, the substrate 110 is provided. Then, a second mask pattern 240 is formed on the substrate 110. The second mask pattern 240 covers the probe cell region (CR) on the substrate 110 and is formed to expose the probe cell isolation region (CIR). Other constituent elements are identical to the above-mentioned first mask pattern 210.

With reference to FIG. 18, the exposed surface of the substrate is etched using the second mask pattern 240 as the etching mask. After the etching, the recess pattern (R) on the surface (s) of the substrate 114 is formed in the probe cell isolation region (CIR) of the substrate 114. Next, the second mask pattern 240 is removed. With reference to FIGS. 11 to 15, subsequent processes are performed to obtain the microarray shown in FIG. 7.

As shown in FIG. 19 optionally, a third mask pattern 250 may be additionally formed after the step of FIG. 18. The third mask pattern 250 is substantially identical with the first mask pattern 210, but it may cover the recess pattern (R) formed at the step of FIG. 18. The third mask pattern 250 should be removed in the subsequent process to produce the microarray 104 of FIG. 7. Meanwhile, if the third mask pattern 250 is not removed but remains to form an isolation layer (reference numeral 220 of FIG. 8) in the subsequent process, a structure substantially identical to that of the microarray 105 of FIG. 8 may be formed. If the third mask pattern 250 is made even so that it can partially remain as an isolation layer (reference numeral 230 of FIG 9) a structure substantially identical to that of the microarray 106 of FIG. 9 may be formed.

Meanwhile, the microarray 105 of FIG. 8 may be formed using, for example, a LOCOS process. Furthermore, the microarray 106 of FIG. 9 may be formed using a shallow trench isolation (STI) process. As the above-mentioned processes are widely known in the related art, a detailed description thereof shall be also omitted.

According to the microarrays obtained from the exemplary embodiments of the present invention, it is possible to couple a large number of probes per unit area because the probes are coupled with the surfaces of the fine particles in either a sphere or an oval. Furthermore, as the fine particles constitute the photonic crystal structure, the detected light intensity increases according to the degree of mixing. Therefore, even with a relatively small amount of integrated probes, mixing can be readily detected.

Having described the exemplary embodiments of the present invention, it is further noted that it is readily apparent to those of reasonable skill in the art that various modifications may be made without departing from the spirit and scope of the invention which is defined by the metes and bounds of the appended claims.

**Claims**

1. A microarray comprising:

   a substrate;
   a plurality of fine particles disposed on the substrate at regular intervals; and
   a plurality probes coupled with the fine particles.

2. The microarray of claim 1, wherein the fine particles are disposed on the substrate to form a single layer.

3. The microarray of claim 2, wherein the fine particles constitute a photonic crystal structure.

4. The microarray of claim 3, wherein a distance between centers of the adjacent fine particles is about 150 to about 20,000 nanometers (nm).

5. The microarray of claim 1, wherein each of the fine particles comprises one of a sphere about 50 to about 10,000 nanometers (nm) in diameter or an oval with a major axis of about 50 to about 10,000 nm.

6. The microarray of claim 1, wherein the fine particles are selected from the group consisting of of methyl methacrylate, styrene, dimethyl siloxane, vinyl alcohol, hydroxy methacrylate, a polymer or copolymer thereof, silicon oxide, and titanium oxide and a combination thereof.

7. The microarray of claim 1, wherein:

   the substrate comprises a plurality of probe cell regions and probe cell isolation regions defining the probe cell regions; and
   the plurality of fine particles are disposed in the probe cell regions.

**8.** The microarray of claim 7, further comprising:

a plurality of mask patterns formed in the probe cell isolation regions.

**9.** The microarray of claim 1, wherein the substrate comprises a recess pattern on the surface thereof.

**10.** A method of producing a microarray, the method comprising:

providing a substrate;
disposing a plurality of fine particles on the substrate so that the fine particles are disposed at regular intervals; and
coupling a plurality probes with the fine particles.

**11.** The method of claim 10, wherein the disposing of the plurality of fine particles on the substrate comprises:

disposing a plurality of fine particle precursors on the substrate; and
reducing sizes of the plurality of fine particle precursors to form the plurality of fine particles disposed at regular intervals.

**12.** The method of claim 11, wherein the disposing of the plurality of fine particle precursors comprises:

preparing a dispersion solution where the plurality of fine particle precursors is dispersed in a solvent; and
performing one of a slit coating or spin coating with the dispersion solution on the substrate.

**13.** The method of claim 11, further comprising:

performing agglomeration of the plurality of fine particle precursors after the plurality of fine particle precursors is disposed.

**14.** The method of claim 13, wherein the performing of the agglomeration of the plurality of fine particle precursors comprises annealing the plurality of fine particle precursors.

**15.** The method of claim 11, wherein the reducing of the sizes of the plurality of fine particle precursors comprises performing isotropy etching of the plurality of fine particle precursors.

**16.** The method of claim 10, further comprising:

activating surfaces of the fine particles to expose functional groups capable of being coupled with the probes before the probes are coupled with the fine particles.

**17.** The method of claim 10, wherein:

the substrate comprises a plurality of probe cell regions and a plurality of probe cell isolation regions defining the probe cell regions; and
the plurality of fine particles is disposed in the probe cell regions.

**18.** The method of claim 17, further comprising:

forming a plurality of mask patterns in the probe cell isolation regions of the substrate,

wherein the plurality of fine particles is disposed in regions defined by the mask patterns of the substrate.

**19.** The method of claim 10, wherein the fine particles are disposed on the substrate to form a single layer.

**20.** The method of claim 19, wherein the fine particles constitute a photonic crystal structure.

**21.** The method of claim 20, wherein a distance between centers of the adjacent fine particles is about 150 to about 20,000 nanometers (nm).

**22.** The method of claim 10, wherein each of the fine particles comprises one of a sphere about 50 to about 10,000 nanometers (nm) in diameter or an oval with a major axis of about 50 to about 10,000 nm.

**23.** The method of claim 10, wherein the fine particles are selected from the group consisting of methyl methacrylate, styrene, dimethyl siloxane, vinyl alcohol, hydroxy methacrylate, a polymer or copolymer thereof, silicon oxide, and titanium oxide and a combination thereof.

**24.** The method of claim 10, wherein the substrate comprises a recess pattern on the surface thereof.

**25.** The microarray of claim 1, wherein each of the probes is coupled to the fine particles via a linker.

# FIG. 1

100

120

122

II

II'

L

CIR    CR

# FIG. 2

100

130   120

110

CR   CIR

# FIG. 3

# FIG. 4

# FIG. 5

102

210    130  120

110

CR    CIR

# FIG. 6

103

R    S    130  120

112

CR    CIR

# FIG. 7

# FIG. 8

# FIG. 9

# FIG. 10

210

110

CR  CIR

# FIG. 11

Heat

120a

210

110

CR  CIR

# FIG. 12

120a

210

110

CR    CIR

# FIG. 13

120a

110

CR    CIR

# FIG. 14

120

110

CR    CIR

# FIG. 15

# FIG. 16

# FIG. 17

# FIG. 18

# FIG. 19

European Patent
Office

## EUROPEAN SEARCH REPORT

Application Number

EP 08 00 2295

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 249 502 A (HITACHI SOFTWARE ENG [JP]) 16 October 2002 (2002-10-16) * abstract * * paragraph [0022] - paragraph [0023] * * paragraph [0034] - paragraph [0038] * * figure 2 * ----- | 1-25 | INV. B01J19/00 C12Q1/68 |
| X | US 6 706 473 B1 (EDMAN CARL FREDERICK [US] ET AL) 16 March 2004 (2004-03-16) * abstract * * column 1, line 20 - line 45 * * column 6, line 53 - column 8, line 13 * * column 17, line 6 - column 18, line 11 * * column 20, line 25 - column 23, line 11 * * column 28, line 1 - column 29, line 5 * * figures 30-40 * ----- | 1-25 | |
| X | US 2004/053354 A1 (IKAWA TAIJI [JP] ET AL) 18 March 2004 (2004-03-18) * abstract * * paragraph [0002] * * paragraph [0039] * * paragraph [0116] - paragraph [0131] * * paragraph [0154] * * figures * ----- | 1-25 | TECHNICAL FIELDS SEARCHED (IPC) B01J C12Q |
| X | WO 01/59432 A (ILLUMINA INC [US]) 16 August 2001 (2001-08-16) * abstract * * page 6, line 3 - page 9, line 22 * * page 11, line 20 - page 13, line 33 * * page 14, line 30 - page 15, line 2 * * page 28, line 16 - line 31 * * claims; figures 1,2 * ----- -/-- | 1-25 | |

The present search report has been drawn up for all claims

| Place of search | Date of completion of the search | Examiner |
|---|---|---|
| Munich | 6 June 2008 | Nazario, Luis |

EPO FORM 1503 03.82 (P04C01)

European Patent
Office

**EUROPEAN SEARCH REPORT**

Application Number

EP 08 00 2295

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 186 659 A (MATSUSHITA ELECTRIC IND CO LTD [JP]) 13 March 2002 (2002-03-13)<br>* abstract *<br>* paragraph [0029] - paragraph [0033] *<br>* paragraph [0039] *<br>* paragraph [0050] - paragraph [0055] *<br>* paragraph [0063] - paragraph [0068] *<br>* paragraph [0106] - paragraph [0120] *<br>* paragraph [0140] - paragraph [0155]; claims; figures 1,2,4,7,8,13 *<br>----- | 1-25 | |
| X | WO 03/020740 A (MOTOROLA INC [US]) 13 March 2003 (2003-03-13)<br>* abstract *<br>* paragraph [0006] - paragraph [0007] *<br>* paragraph [0025] - paragraph [0027] *<br>* paragraph [0029] - paragraph [0033] *<br>* paragraph [0037] - paragraph [0040]; claims; figure 2 *<br>----- | 1-25 | |

TECHNICAL FIELDS
SEARCHED    (IPC)

The present search report has been drawn up for all claims

| Place of search | Date of completion of the search | Examiner |
|---|---|---|
| Munich | 6 June 2008 | Nazario, Luis |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

......................................................................................

& : member of the same patent family, corresponding
    document

EPO FORM 1503 03.82 (P04C01)

**EP 1 958 689 A1**

## ANNEX TO THE EUROPEAN SEARCH REPORT
## ON EUROPEAN PATENT APPLICATION NO.

EP 08 00 2295

This annex lists the patent family members relating to the patent documents cited in the above-mentioned European search report.
The members are as contained in the European Patent Office EDP file on
The European Patent Office is in no way liable for these particulars which are merely given for the purpose of information.

06-06-2008

| Patent document cited in search report | | Publication date | Patent family member(s) | | Publication date |
|---|---|---|---|---|---|
| EP 1249502 | A | 16-10-2002 | JP | 2002311027 A | 23-10-2002 |
| | | | US | 2003190628 A1 | 09-10-2003 |
| US 6706473 | B1 | 16-03-2004 | NONE | | |
| US 2004053354 | A1 | 18-03-2004 | JP | 3818265 B2 | 06-09-2006 |
| | | | JP | 2003329682 A | 19-11-2003 |
| WO 0159432 | A | 16-08-2001 | AU | 3976001 A | 20-08-2001 |
| | | | AU | 2001239760 B2 | 24-11-2005 |
| | | | CA | 2399908 A1 | 16-08-2001 |
| | | | EP | 1257805 A2 | 20-11-2002 |
| | | | JP | 2003522969 T | 29-07-2003 |
| EP 1186659 | A | 13-03-2002 | AU | 4116401 A | 24-09-2001 |
| | | | WO | 0168834 A1 | 20-09-2001 |
| | | | US | 2003013096 A1 | 16-01-2003 |
| WO 03020740 | A | 13-03-2003 | US | 2003044801 A1 | 06-03-2003 |

EPO FORM P0459

For more details about this annex : see Official Journal of the European Patent Office, No. 12/82

19